(19) **Europäisches Patentamt / European Patent Office / Office européen des brevets**

(11) **EP 4 699 563 A1**

(12) **EUROPEAN PATENT APPLICATION**

(43) Date of publication:
25.02.2026 Bulletin 2026/09

(51) International Patent Classification (IPC):
*A61B 34/30* (2016.01)    *A61B 34/20* (2016.01)
*A61B 90/00* (2016.01)

(21) Application number: 25190143.5

(22) Date of filing: 17.07.2025

(52) Cooperative Patent Classification (CPC):
**A61B 34/30;** A61B 2034/2055; A61B 2034/304;
A61B 2090/064

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 20.08.2024  US 202463684874 P
16.01.2025  TW 114101931

(71) Applicant: **National Taiwan University**
**Taipei, 10617 (TW)**

(72) Inventors:
• YEN, Ping-Lang
10617 Taipei (TW)
• ZUO, Hao-Cheng
10617 Taipei (TW)
• CHUNG, Cheng-Yen
10617 Taipei (TW)

(74) Representative: **Winter, Brandl - Partnerschaft
mbB
Alois-Steinecker-Straße 22
85354 Freising (DE)**

(54) **COMPOUND ROBOTIC SYSTEM**

(57)    A compound robotic system includes a first robot, a second robot, a needle insertion device, a first optical positioning ball module, plural skin markers, an optical tracker, a shared controller and a computer calculation unit. The volume and movement range of the second robot are respectively less than the volume and movement range of the first robot, and the positioning accuracy of the second robot is greater than the positioning accuracy of the first robot. The first optical positioning ball module is located on the second robot. The optical tracker is configured to measure the first optical positioning ball module to obtain a position of the second robot, and to measure the skin markers to estimate a planning path. The shared controller is configured to adjust a collaborative rate of the first robot to the second robot by regulating a shared weighting.

FIG. 1

## Description

## BACKGROUND

Technical field

The present disclosure relates to a compound robotic system, and particularly relates to a surgical compound robotic system.

Description of Related Art

During a minimally invasive surgery, the lesion cannot be directly seen, and during the surgery, additional CT (computed tomography) images are required to guide the doctor to place a puncture needle on a puncture path planned before the surgery. In order to ensure the safety and accuracy of puncturing, during the surgery, confirmation is performed by the computer calculation unitized tomography several times, which will cause a lot of radiation damage to both the doctor and the patient.

During the surgery, the positions of internal organs and lesions are different from those on the CT images due to the patient's respiratory motion or the extrusion of the tissues caused by the puncture needle. Therefore, it is easy to make mistakes to guide the doctor to perform the puncture surgery by the CT images alone; and the puncture needle when passing through the fascia is prone to deviation. At present, the deviation of a needle body is clinically corrected by withdrawing the puncture needle and re-inserting the needle, which causes additional damage to the patient and is time-consuming.

## SUMMARY

According to some embodiments of the present disclosure, a compound robotic system comprises a first robot, a second robot, a needle insertion device, a first optical positioning ball module, plural skin markers, an optical tracker, a shared controller, and a computer calculation unit. The first robot is located on a carrying platform. The second robot is located at one end of the first robot away from the carrying platform, wherein the volume and movement range of the second robot are respectively less than the volume and movement range of the first robot, and the positioning accuracy of the second robot is greater than the positioning accuracy of the first robot. The needle insertion device is located on the second robot. The first optical positioning ball module is located on the second robot. The skin markers are located on a skin surface of the patient. The optical tracker is configured to measure the first optical positioning ball module to obtain a position of the second robot, and to measure the skin markers to estimate a planning path. The shared controller is electrically connected to the first robot and the second robot and configured to adjust a collaborative rate of the first robot to the second robot by regulating a shared weighting according to the position of the second robot and the planning path, and the computer calculation unit is electrically connected to the first robot, the second robot, the needle insertion device and the optical tracker.

In some embodiments, the needle insertion device comprises a guide slot, a puncture needle, and a sensor. The puncture needle is located in the guide slot, and the sensor is configured to sense the force and tactile feedback of the puncture needle when passing through a muscle tissue of the patient.

In some embodiments, the second robot comprises a base, a moving platform, and plural moving links. The base is connected to one end of the first robot away from the carrying platform, wherein the first robot is configured to control a position of the base. The moving platform is connected to the needle insertion device and the first optical positioning ball module, and the moving links are located between the base and the moving platform and each have plural joints so that the second robot has plural degrees of freedom.

In some embodiments, the second robot is configured to control an entry attitude of the needle insertion device, and the shared controller is configured to share a target attitude of the first robot and a target attitude of the second robot.

In some embodiments, the target attitude of the first robot conforms to the following formula:

$$\left( {}^{Path}_{B_d}\boldsymbol{X} \right) = {}^{Path}_{B}\boldsymbol{X} + w \left( {}^{Path}_{t_d}\boldsymbol{X} - {}^{Path}_{t}\boldsymbol{X} \right)$$

wherein ${}^{Path}_{t_d}\boldsymbol{X}$ is a target attitude of the puncture needle of the needle insertion device relative to the planning path, ${}^{Path}_{t}\boldsymbol{X}$ is a current attitude of the puncture needle relative to the planning path, ${}^{Path}_{B}\boldsymbol{X}$ is a current attitude of the base of the second robot relative to the planning path, $_{B_d}^{Path}X$ is a target attitude of the base of the second robot relative to the planning path, and w is the shared weighting.

In some embodiments, the target attitude of the second robot conforms to the following formula:

$$_{t_d}^{B_d}T = {_{B_d}^{path}T}^{-1}\,{_{t_d}^{Path}T}$$

wherein $_{B_d}^{path}T$ is a target attitude to be achieved by the base of the second robot relative to the planning path, $_{B_d}^{path}T^{-1}$ is an inverse matrix of $_{B_d}^{path}T$, and $_{t_d}^{B_d}T$ is an attitude of the needle insertion device relative to the base when the second robot completes alignment of an entry path with the planning path.

In some embodiments, the target attitude of the first robot conforms to the following formula:

$$_{\boldsymbol{B_d}}^{Path}T = {_{t}^{Path}T}\,{_{B_d}^{t}T}$$

wherein $_{B_d}^{Path}T$ is the target attitude of the first robot and $_{t}^{Path}T$ is a current attitude of the puncture needle relative to the planning path, and the target attitude $_{B_d}^{Path}T$ of the first robot is planned according to the current attitude $_{t}^{Path}T$ of the puncture needle and a working space of the second robot, so that $_{B_d}^{t}T$ meets the requirements of the working space of the second robot.

In some embodiments, the target attitude of the second robot conforms to the following formula:

$$_{t_d}^{B}T = {_{B}^{path}T}^{-1}\,{_{t_d}^{Path}T}$$

wherein $_{t_d}^{B}T$ is the target attitude of the second robot, $_{B}^{path}T^{-1}$ is an inverse matrix of $_{B}^{path}T$, $_{B}^{path}T$ is a current attitude of the base of the second robot relative to the planning path, and $_{t_d}^{Path}T$ is an attitude of the needle insertion device relative to the planning path when the second robot completes alignment of the entry path with the planning path.

In some embodiments, the shared controller is configured to adjust the shared weighting according to a tracking error of the optical tracker for the first optical positioning ball module.

In some embodiments, the compound robotic system further comprises a second optical positioning ball module located on the first robot, wherein the optical tracker is configured to sense the second optical positioning ball module and then to obtain a reference coordinate.

In some embodiments, the optical tracker is configured to measure movement of the skin markers to estimate a respiratory motion of the patient. The computer calculation unit is configured to create a respiratory model based on the respiratory motion, and the respiratory model is configured to estimate an instant position of a lesion of the patient.

In some embodiments, the computer calculation unit is configured to obtain the reference coordinate from the second optical positioning ball module and to filter out the respiratory motion of the patient to track a hard tissue motion of the patient.

In some embodiments, the hard tissue motion of the patient obtains a coordinate of movement of the patient by means of an iterative closest point (ICP).

In some embodiments, the respiratory model conforms to the following formula:

$$\begin{bmatrix} x_i(t) \\ y_i(t) \\ z_i(t) \end{bmatrix} = \begin{bmatrix} a_1 x_i(t-1) + a_2 x_i(t-2) + \cdots + a_k x_i(t-k) \\ b_1 y_i(t-1) + b_2 y_i(t-2) + \cdots + b_k y_i(t-k) \\ c_1 z_i(t-1) + c_2 z_i(t-2) + \cdots + c_k z_i(t-k) \end{bmatrix}$$

wherein $i$ is an index of the skin marker, $x_i$, $y_i$ and $z_i$ are x, y and z coordinates of the skin marker respectively, t is a time, k is the number of past moments used, and $a_1$-$a_k$, $b_1$-$b_k$ and $c_1$-$c_k$ are coefficients calculated from an autoregression model.

In some embodiments, the respiratory model is configured to instantly estimate positions of the skin markers for the next time.

In some embodiments, the respiratory motion of the skin markers outside and a position of the lesion inside are configured to create an outside-inside correlation model to estimate the instant position of the lesion, the outside-inside correlation model being calculated using a linear regression.

According to some embodiments of the present disclosure, a compound robotic system comprises a first robot, a second robot, a needle insertion device, a first optical positioning ball module, plural skin markers, an optical tracker, a shared controller, and a computer calculation unit. The first robot is located on a carrying platform. The second robot is located at one end of the first robot away from the carrying platform. The needle insertion device is located on the second robot and comprises a guide slot, a puncture needle and a sensor, wherein the puncture needle is located in the guide slot, and the sensor is configured to sense the force and tactile feedback of the puncture needle when passing through a muscle tissue of a patient. The first optical positioning ball module is located on the second robot. The skin markers are located on a skin surface of the patient. The optical tracker is configured to measure the first optical positioning ball module to obtain a position of the second robot, and to measure the skin markers to estimate a planning path. The shared controller is electrically connected to the first robot and the second robot, and configured to adjust a collaborative rate of the first robot to the second robot by regulating a shared weighting according to the position of the second robot and the planning path, and the computer calculation unit is electrically connected to the first robot, the second robot, the needle insertion device and the optical tracker.

In some embodiments, the second robot comprises a base, a moving platform, and plural moving links. The base is connected to one end of the first robot away from the carrying platform, wherein the first robot is configured to control a position of the base. The moving platform is connected to the needle insertion device and the first optical positioning ball module, and the moving links are located between the base and the moving platform and each have plural joints so that the second robot has plural degrees of freedom.

In some embodiments, the second robot is configured to control an entry attitude of the needle insertion device, and the shared controller is configured to share a target attitude of the first robot and a target attitude of the second robot.

In some embodiments, the compound robotic system further comprises a second optical positioning ball module located on the first robot, wherein the optical tracker is configured to sense the second optical positioning ball module and then to obtain a reference coordinate.

In some embodiments, the optical tracker is configured to measure movement of the skin markers to estimate a respiratory motion of the patient. The computer calculation unit is configured to create a respiratory model based on the respiratory motion, and the respiratory model is configured to estimate an instant position of a lesion of the patient.

In some embodiments, the computer calculation unit is configured to obtain the reference coordinate from the second optical positioning ball module and to filter out the respiratory motion of the patient to track a hard tissue motion of the patient.

In the above embodiments of the present disclosure, the plural skin markers of the compound robotic system are located on the skin surface corresponding to the lesion, and the skin markers are measured by the optical tracker to indirectly estimate the position of the lesion in a non-invasive manner so that the risks and discomforts of the patient previously brought about by traditional invasive positioning methods are reduced. Moreover, the optical tracker can obtain the position of the second robot by measuring the first optical positioning ball module. The computer calculation unit can further calculate the planning path through skin marker positioning information and second robot positioning information returned by the optical tracker and transmit the planning path to the shared controller so that the first robot, the second robot and the needle insertion device further achieve the target postures to guide the doctor to place the puncture needle on the planning path. The target attitude of the needle insertion device is controlled by a combination of a large range movement of the first robot and a small range movement of the second robot, so that a positioning process improves the positioning accuracy required for the planning path. In this way, the accuracy of puncturing and the success rate of the surgery can be improved.

**BRIEF DESCRIPTION OF THE DRAWINGS**

FIG. 1 is a stereogram of a compound robotic system according to one embodiment of the present embodiment;

FIG. 2 is an enlarged view of a second robot and a needle insertion device in FIG. 1;

FIG. 3A is a schematic diagram of a collaborative rate of a first robot to the second robot in FIG. 1;

FIG. 3B is another schematic diagram of the collaborative rate of the first robot to the second robot in FIG. 1

FIG. 4 is a block diagram showing the first robot, the second robot and the shared controller in FIG. 3A;

FIG. 5 is a stereogram of the compound robotic system in FIG. 1 from another view angle;

FIG. 6 is a schematic diagram showing an optical tracker, skin markers, and a second optical positioning ball module when in use in FIG. 1, in which the first robot, the second robot, and the needle insertion device are omitted;

FIG. 7 is a time-varying periodogram of a respiratory model according to one embodiment of the present disclosure;

FIG. 8 is a schematic diagram of point set alignment calculated by movement of a patient by means of an iterative closest point (ICP) according to one embodiment of the present disclosure; and

FIG. 9 is a schematic diagram showing motions of the skin markers and lesions according to one embodiment of the present disclosure.

## DETAILED DESCRIPTION

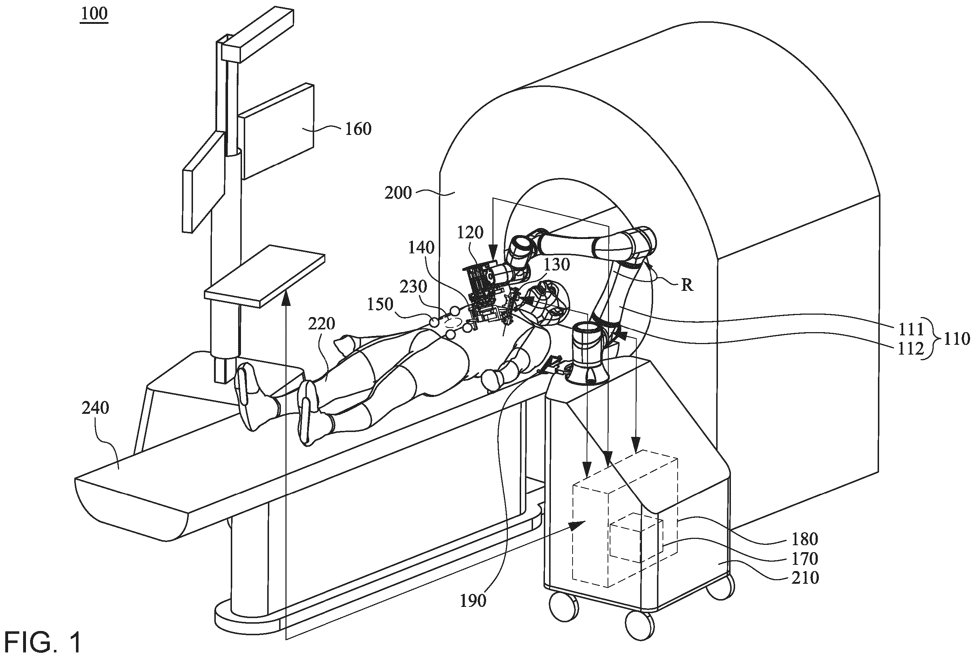

FIG. 1 is a stereogram of a compound robotic system 100 according to one embodiment of the present embodiment. The compound robotic system 100 includes a first robot 110, a second robot 120, a needle insertion device 130, a first optical positioning ball module 140, plural skin markers 150, an optical tracker 160, a shared controller 170, and a computer calculation unit 180. The first robot 110 is located on a carrying platform 210, and in some embodiments, the shared controller 170 and the computer calculation unit 180 are provided in the carrying platform 210. The first robot 110 is a serial Macro robot. The first robot 110 has plural links 111, the links 111 are connected in series and a joint 112 is provided between two links 111, so that the first robot 110 has plural rotating directions R to adjust attitudes. The second robot 120 is a parallel Mini robot. The second robot 120 is located at one end of the first robot 110 away from the carrying platform 210, where the volume and movement range of the second robot 120 are respectively less than the volume and movement range of the first robot 110. The needle insertion device 130 is located on the second robot 120.

The first optical positioning ball module 140 is located on the second robot 120. The skin markers 150 are located on a skin surface of a patient 220, and a position of the skin surface is an external skin position corresponding to an in vivo lesion 230 (e.g., tumor). The optical tracker 160 is configured to measure the first optical positioning ball module 140 to obtain a position of the second robot 120 and to measure the skin markers 150 to indirectly track a position of the lesion 230. In some embodiments, the optical tracker 160 can be further combined with medical imaging equipment such as a computed tomography (CT) and an ultrasound. The optical tracker 160 tracks the lesion 230 of the patient by the first optical positioning ball module 140 to realize dynamic tracking of spatial positions of the compound robotic system 100 and the lesion 230.

In some embodiments, the shared controller 170 is provided in the computer calculation unit 180. The computer calculation unit 180 is electrically connected to the first robot 110, the second robot 120, the needle insertion device 130 and the optical tracker 160, and the shared controller 170 is electrically connected to the first robot 110 and the second robot 120.

In the following description, the design of the second robot 120 and the needle insertion device 130 will be described in detail.

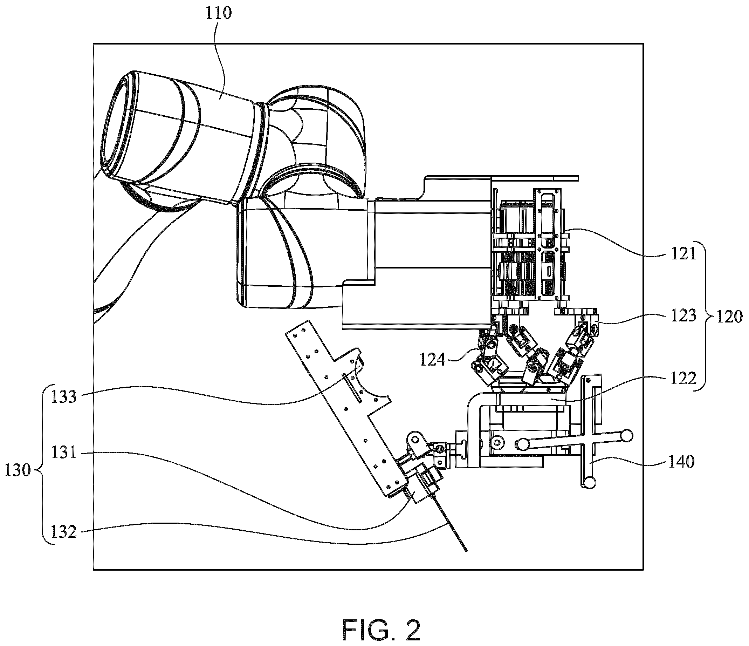

FIG. 2 is an enlarged view of the second robot 120 and the needle insertion device 130 in FIG. 1. The needle insertion device 130 includes a guide slot 131, a puncture needle 132 and a sensor 133, where the puncture needle 132 is located in the guide slot 131, and the guide slot 131 can guide a needle insertion direction of the puncture needle 132 and loosen or grab back the puncture needle 132 as required in a puncture stage. The sensor 133 is configured to sense the force and tactile feedback of the puncture needle 132 when passing through a muscle tissue of the patient 220 (see FIG. 1) and to sense whether the puncture needle 132 punctures the lesion 230 (see FIG. 1).

The second robot 120 includes a base 121, a moving platform 122 and plural moving links 123. The base 121 is connected to one end of the first robot 110 away from the carrying platform 210, where the first robot 110 is configured to control a position of the base 121. The moving platform 122 is connected to the needle insertion device 130 and the first optical positioning ball module 140. The moving links 123 are located between the base 121 and the moving platform 122. In addition, each of the moving links 123 has plural joints 124. The number of the moving links 123 is six, but not limited to this. From the visual angle of FIG. 2, the other three moving links 123 are obscured. The moving links 123 are designed so that the second robot 120 has plural degrees of freedom (e.g., six degrees of freedom) and can thus adjust the moving links 123 more subtly, and the positioning accuracy of the second robot 120 is greater than that of the first robot 110.

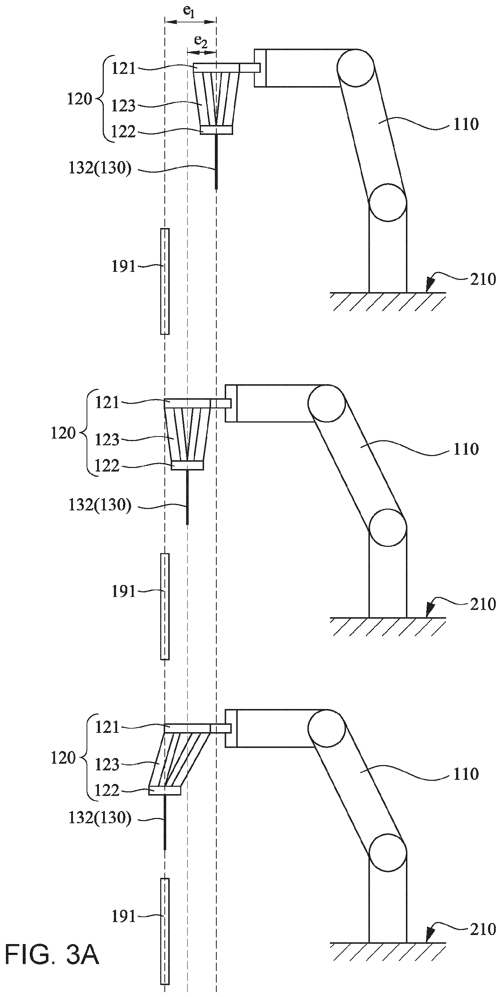

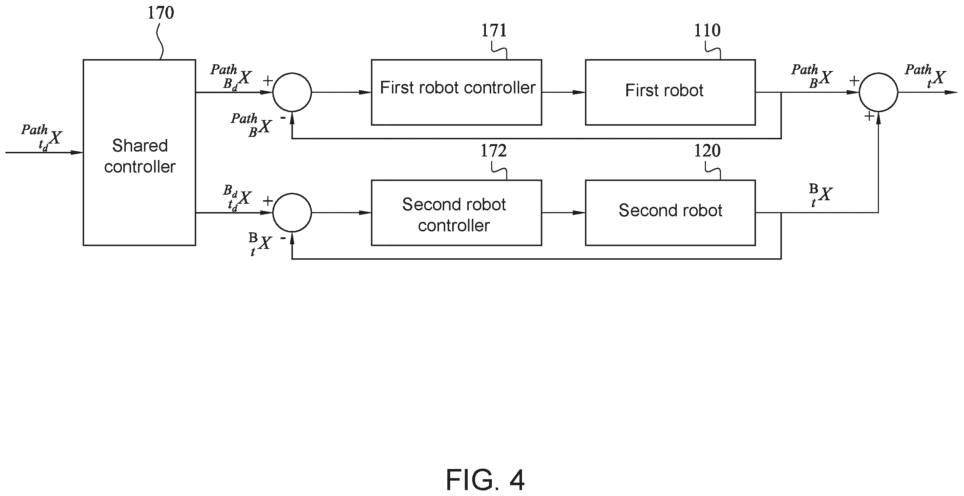

FIG. 3A is a schematic diagram of a collaborative rate of the first robot 110 to the second robot 120 in FIG. 1. FIG. 4 is a block diagram showing the first robot 110, the second robot 120 and the shared controller 170 in FIG. 3A. Referring to FIG. 3A and FIG. 4, the first robot 110 is configured to control the base 121 of the second robot 120, and the second robot 120 is configured to control an entry attitude (position and orientation) of the needle insertion device 130. The shared controller 170 further includes a first robot controller 171 and a second robot controller 172 and is configured to share a target attitude of the first robot 110 and a target attitude of the second robot 120, respectively. The first robot controller 171 is electrically connected to the first robot 110, and the second robot controller 172 is electrically connected to the second robot 120. The computer calculation unit 180 (see FIG. 1) estimates a planning path 191 of a surgery according to the position of the second robot 120 and the position of the lesion 230 returned by the optical tracker 160 (see FIG. 1). The shared controller 170 is configured to adjust a shared weighting according to a tracking error of the optical tracker 160 for the first optical positioning ball module 140 (see FIG. 2), where the tracking error is shown in FIG. 3A as a base 121 attitude error $V_2$ between a current attitude of the second robot 120 and a target attitude of the planning path 191 and a puncture needle 132 attitude error $V_1$ between a current attitude of the needle insertion device 130 and the target attitude of the planning path 191, based on which the shared weighting is regulated to adjust the collaborative rate of the first robot 110 to the second robot 120. The attitudes are the positions and orientations of the first robot 110, the second robot 120 and the needle insertion device 130. The attitude can be expressed by a transformation matrix T, and also can be expressed by a 6x1 vector X, in which the first three components are positions and the last three are orientation angles.

In the present embodiment, the target attitude of the first robot 110 conforms to the following formula:

$$\begin{pmatrix} ^{Path}_{B_d}\boldsymbol{X} \end{pmatrix} = ^{Path}_{B}\boldsymbol{X} + w \begin{pmatrix} ^{Path}_{t_d}\boldsymbol{X} - ^{Path}_{t}\boldsymbol{X} \end{pmatrix}$$

wherein $^{Path}_{t_d}\boldsymbol{X}$ is a target attitude of the puncture needle 132 of the needle insertion device 130 relative to the planning path 191, $^{Path}_{t}\boldsymbol{X}$ is a current attitude of the puncture needle 132 relative to the planning path 191, $^{Path}_{B}\boldsymbol{X}$ is a current attitude of the base 121 of the second robot 120 relative to the planning path 191, $^{Path}_{B_d}\boldsymbol{X}$ is a target attitude of the base 121 of the second robot 120 relative to the planning path 191, and w is the shared weighting. For example, the shared weighting can be a fixed value between 0 and 1. When the tracking error is greater than the movement range of the second robot 120, the shared weighting is 1, which is completely controlled by the first robot 110; when the tracking error is small enough, the shared weighting is 0, which is completely controlled by the second robot 120; and when the tracking error is between the above two, the shared weighting will be exponentially related to the value of the error, and as the error becomes smaller, the control of the second robot 120 is increased gradually.

In addition, the target attitude of the second robot 120 conforms to the following formula:

$$^{B_d}_{t_d}\boldsymbol{T} = ^{path}_{B_d}\boldsymbol{T}^{-1}\,^{Path}_{t_d}\boldsymbol{T}$$

where $^{path}_{B_d}\boldsymbol{T}$ is a target attitude to be achieved by the base 121 of the second robot 120 relative to the planning path 191, $^{path}_{B_d}\boldsymbol{T}^{-1}$ is an inverse matrix of $^{path}_{B_d}\boldsymbol{T}$, and $^{B_d}_{t_d}\boldsymbol{T}$ is an attitude of the needle insertion device 130 relative to the base 121 when the second robot 120 completes alignment of an entry path with the planning path 191.

Referring to FIG. 4, the first robot 110 is configured to control the base 121 of the second robot 120, the second robot 120 is configured to control the entry attitude (position and orientation) of the needle insertion device 130, and the shared controller 170 is used to share the target attitudes of the first robot 110 and the second robot. First, information of a target attitude required by the puncture needle 132 of the needle insertion device 130 will be transmitted to the shared controller 170. In order to achieve the target attitude, the first robot 110 needs to be controlled by the first robot controller 171 to adjust a current attitude of the base 121 of the second robot 120 so that the base 121 of the second robot 120 achieves the target attitude, and the second robot 120 is controlled by the second robot controller 172 to adjust an integrated current attitude of the puncture needle 132 of the needle insertion device 130 and the base 121 of the second robot 120 so that the puncture needle 132 of the needle insertion device 130 and the base 121 of the second robot 120 achieve the integrated target attitude.

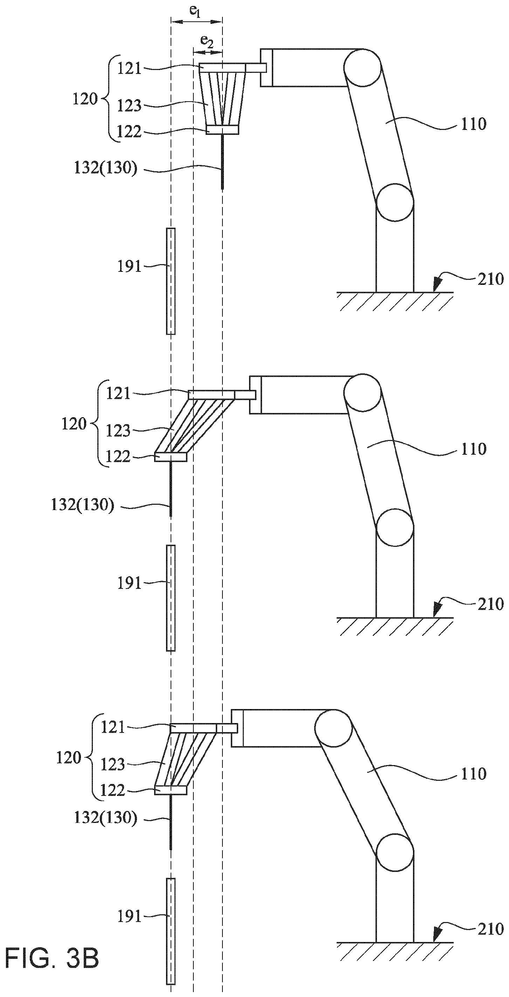

FIG. 3B is another schematic diagram of the collaborative rate of the first robot 110 to the second robot 120 in FIG. 1. The target attitude of the second robot 120 conforms to the following formula:

$$_{t_d}^{B}T = {}_{B}^{path}T^{-1}\,{}_{t_d}^{Path}T$$

where $_{t_d}^{B}T$ is the target attitude of the second robot 120, which is calculated according to the above formula. $_{B}^{path}T^{-1}$ is an inverse matrix of $_{B}^{path}T$, $_{B}^{path}T$ is a current attitude of the base 121 of the second robot 120 relative to the planning path 191, $_{t_d}^{Path}T$ is an attitude of the needle insertion device 130 relative to the planning path 191 when the second robot 120 completes alignment of the entry path with the planning path 191. In addition, the target attitude of the first robot 110 conforms to the following formula:

$$_{B_d}^{Path}T = {}_{t}^{Path}T\,{}_{B_d}^{t}T$$

where $_{B_d}^{Path}T$ is the target attitude of the first robot 110, which is calculated according to the above formula. where $_{t}^{Path}T$ is the current attitude of the puncture needle 132 relative to the planning path 191, and the target attitude $_{B_d}^{Path}T$ of the first robot 110 is planned according to the current attitude $_{t}^{Path}T$ of the puncture needle 132 and a working space of the second robot 120, so that $_{B_d}^{t}T$ meet the requirements of the working space of the second robot 120.

The embodiment of FIG. 3A differs from the embodiment of FIG. 3B in that in the embodiment of FIG. 3A, the target attitude of the first robot 110 and the target attitude of the second robot 120 are configured based on the rate according to an error between the position of the second robot 120 and the planning path 191 and shared to the first robot 110 and the second robot 120 to compensate. In the embodiment of FIG. 3B, the second robot 120 is responsible for error compensation of the position of the puncture needle 132 and the planning path 191, while the first robot 110 is responsible for moving the base 121 of the second robot 120 to maintain the attitude of the second robot 120 in the working space.

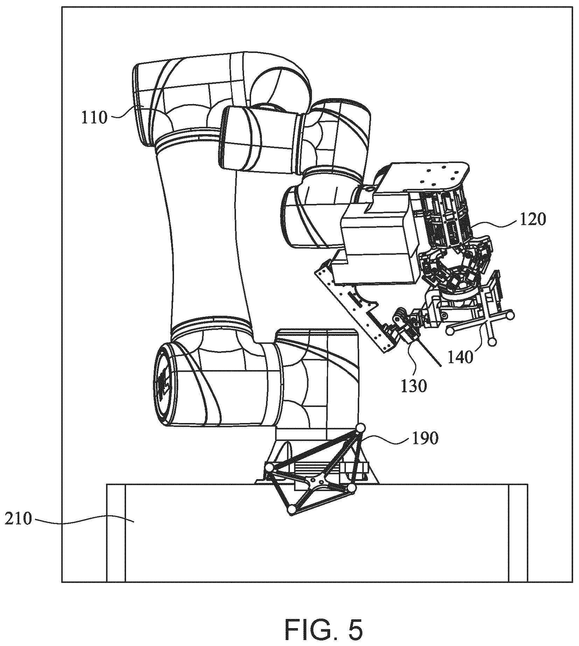

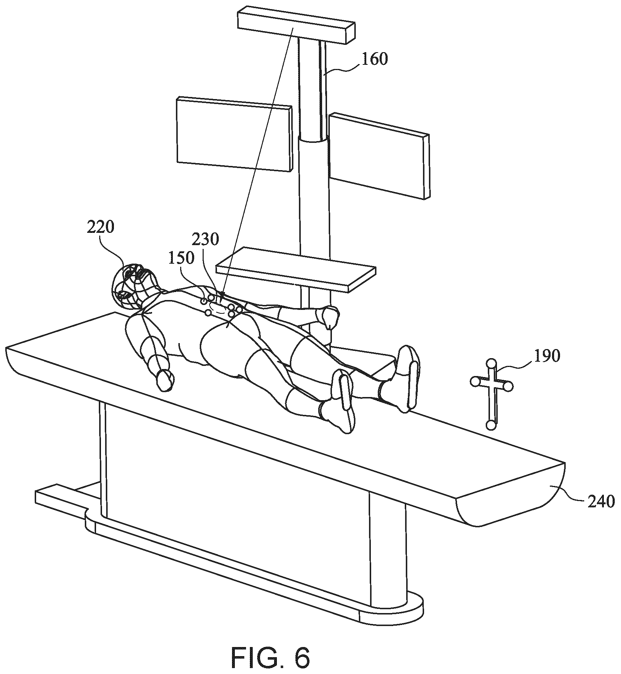

FIG. 5 is a stereogram of the compound robotic system 100 in FIG. 1 from another view angle. FIG. 6 is a schematic diagram showing the optical tracker 160, the skin markers 150, and the second optical positioning ball module 190 when in use in FIG. 1, in which the first robot 110, the second robot 120, and the needle insertion device 130 are omitted. Referring to FIG. 5 and FIG. 6, the compound robotic system 100 further includes a second optical positioning ball module 190. The second optical positioning ball module 190 is located on the first robot 110, where the optical tracker 160 is configured to sense the second optical positioning ball module 190 and then to obtain a reference coordinate. In FIG. 6, for clear presentation, the first robot 110 and the second robot 120 (see FIG. 1) are omitted, and only the second optical positioning ball module 190 is shown. To avoid obtaining coordinates of the patient 220 using invasive methods (e.g., marking by placing a bone pin on the bone), non-invasive skin markers150 are placed on a skin surface corresponding to the position of the lesion 230, and the optical tracker 160 is configured to measure movement of the skin markers 150 as the skin surface expands and deforms with the fluctuation of the patient's respiration to estimate a respiratory motion of the patient 220 without a hard tissue motion.

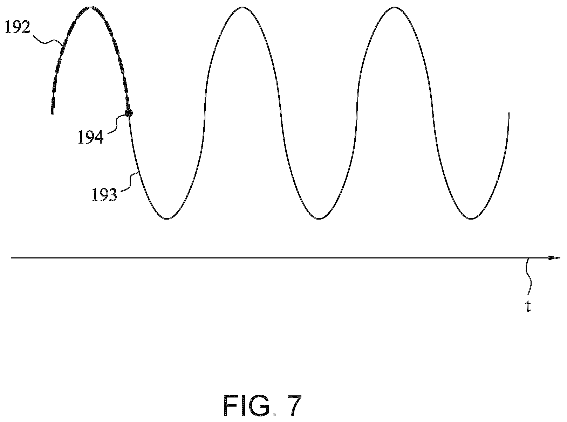

FIG. 7 is a time-varying periodogram of a respiratory model according to one embodiment of the present disclosure. The computer calculation unit 180 (see Figure 1) is configured to create the respiratory model according to a periodic respiratory motion varying in time t. A dotted line 192 and a solid line 193 are the respiratory motions, where the dotted line 192 is a segment of the solid line 193, which represents measured input respiratory information. A predicted output value 194 for the next time is predicted by the input respiratory information of the dotted line 192, and the respiratory model is configured to estimate an instant position of the lesion 230 of the patient 220 (see FIG. 6). The respiratory model conforms to the following formula:

$$\begin{bmatrix} x_i(t) \\ y_i(t) \\ z_i(t) \end{bmatrix} = \begin{bmatrix} a_1 x_i(t-1) + a_2 x_i(t-2) + \cdots + a_k x_i(t-k) \\ b_1 y_i(t-1) + b_2 y_i(t-2) + \cdots + b_k y_i(t-k) \\ c_1 z_i(t-1) + c_2 z_i(t-2) + \cdots + c_k z_i(t-k) \end{bmatrix}$$

wherein $i$ is an index of the skin marker 150, $x_i$, $y_i$ and $z_i$ are x, y and z coordinates of the skin marker 150 respectively, t is a time, k is the number of past moments used, and $a_1$-$a_k$, $b_1$-$b_k$ and $c_1$-$c_k$ are coefficients calculated from an autoregression model (AR model). Data of the respiratory motion is essentially time series data, and has strong autocorrelation, so the autoregression model is suitable for predicting the respiratory motion, which also means that past data can be effectively used to predict future states. Therefore, the respiratory model is configured to instantly estimate a position of the skin marker 150 for the next time by using the respiratory information at the past k moment.

In the present embodiment, the respiratory model can further be updated regularly. Such updating strategy can not only improve the adaptability of the model, for example, in the face of unstable respiratory data, the model better reflects the patient's respiration state over time, but also effectively reduce a prediction deviation caused by the respiratory model being fixed for a long time.

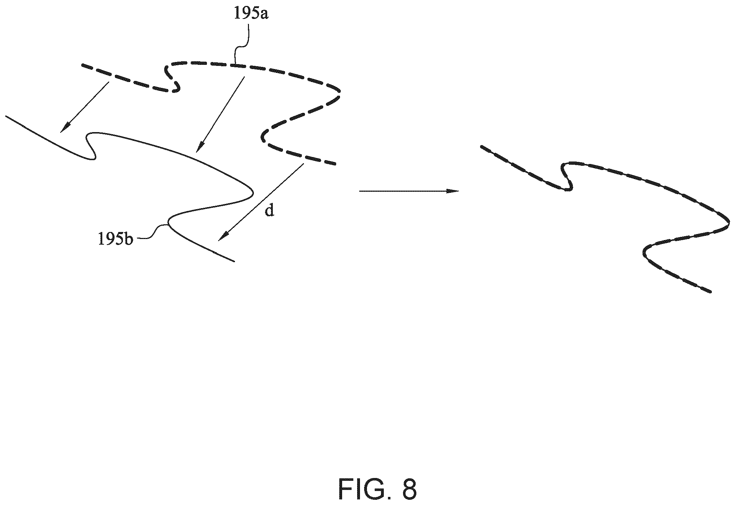

FIG. 8 is a schematic diagram of point set alignment calculated by movement of the patient 220 by means of an iterative closest point (ICP) according to one embodiment of the present disclosure. A solid line 195b represents information on points of n skin markers 150 (see FIG. 6) when the patient 220 has no hard tissue motion, and a dotted line 195a represents information on points of n skin markers 150 after the patient 220 has a movement of the hard tissue motion. Basically, since the hard tissue motion of the patient 220 is to change a relative coordinate position, the solid line 195b and the dotted line 195a have nearly the same shape. The hard tissue motion of the patient 220 can obtain a coordinate of movement of the patient 220 by means of an iterative closest point (ICP). The iterative closest point (ICP) is an algorithm used for 3D alignment (or point cloud alignment). A main goal is to find a best alignment between two sets of point clouds in a 3D space (the two sets of point clouds are respectively the dotted line 195a and the solid line 195b shown in the figure) to minimize a distance d between the two sets of point clouds. The computer calculation unit 180 (see Figure 1) is configured to obtain a reference coordinate with the second optical positioning ball module 190 (see FIG. 6) and to further filter out the respiratory motion of the patient 220 to track the hard tissue motion of the patient 220.

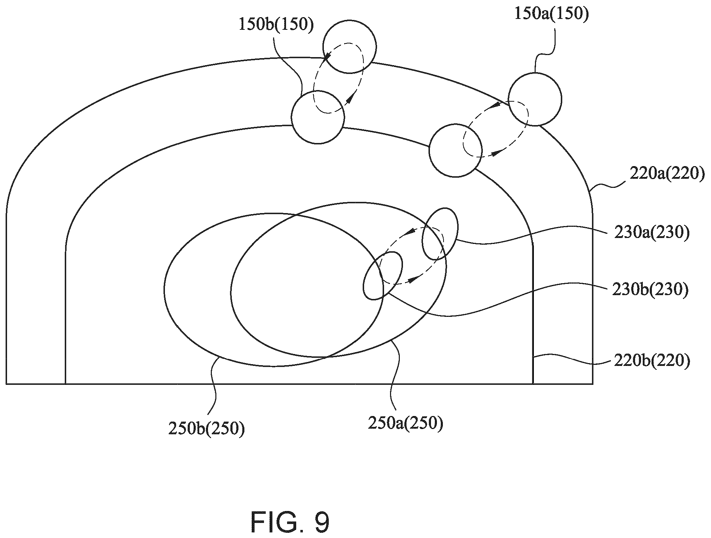

FIG. 9 is a schematic diagram showing motions of the skin markers 150 and the lesion 230 according to one embodiment of the present disclosure. As shown, a skin marker 150a, a patient 220a, a lesion 230a and an organ 250a are position states of the skin marker 150, the patient 220, the lesion 230 and the organ 250 during an inspiration, respectively; a skin marker 150b, a patient 220b, a lesion 230b, and an organ 250b are position states of the skin marker 150, the patient 220, the lesion 230, and the organ 250 during an expiration, respectively; and the inspiration and the expiration will alternately occur in the respiratory motion as time changes. The movements, in an alternating relationship, of the position of the lesion 230a during the inspiration and the position of the lesion 230b during the expiration are, during respiration, generally due to the attachment of the lesion to the position of the organ 250a during the inspiration and the position of the organ 250b during the expiration, such as the liver or kidney, which produce an alternating movement relationship under the influence of respiration. The organs 250 will move up and down in response to a motion of a diaphragm during respiration, so the lesion 230 attached to the organs 250 moves accordingly. An outside-inside correlation model is created based on the configuration of the respiratory motion of the skin markers 150 outside and the position of the lesion 230 inside to estimate an instant position of the lesion 230, where the outside-inside correlation model is calculated using a linear regression. The model can instantly estimate dynamic position changes of the lesion 230a during an inspiration and the lesion 230b during an expiration caused by motions such as respiration by taking a non-invasive alternate relationship between the position of the skin marker 150a during the inspiration and the position of the skin marker 150b during the expiration as a measurement value. The outside-inside correlation model conforms to the following formula:

$$\begin{bmatrix} x_{tumor} \\ y_{tumor} \\ z_{tumor} \end{bmatrix} = \sum_{i=1}^{n} \begin{bmatrix} a_i \\ b_i \\ c_i \end{bmatrix} x_{SM,i} + \sum_{i=1}^{n} \begin{bmatrix} d_i \\ e_i \\ f_i \end{bmatrix} y_{SM,i} + \sum_{i=1}^{n} \begin{bmatrix} g_i \\ h_i \\ i_i \end{bmatrix} z_{SM,i} + \begin{bmatrix} j_i \\ k_i \\ l_i \end{bmatrix}$$

where n is the number of the skin markers 150; and $a_i \sim l_i$ are model parameters calculated using the linear regression. The formula uses three-direction movement of the time-varying positions of the plural skin markers 150a during the inspiration and the skin markers 150b during the expiration to perform linear regression calculation to learn the changes in the relationship between the internal lesion 230 and the external skin marker 150 with the respiratory motion. After training is completed, the correlation model can be used to predict a new input position.

**Claims**

1. A compound robotic system, comprising:

   a first robot (110), located on a carrying platform;
   a second robot (120), located at one end of the first robot (110) away from the carrying platform, wherein a volume and a movement range of the second robot (120) are respectively less than a volume and a movement range of the first robot (110), and a positioning accuracy of the second robot (120) is greater than a positioning accuracy of the first robot (110);

a needle insertion device (130), located on the second robot (120);

a first optical positioning ball module (140), located on the second robot (120);

a plurality of skin markers (150), located on a skin surface of a patient;

an optical tracker (160), configured to measure the first optical positioning ball module (140) to obtain a position of the second robot (120), and to measure the skin markers (150) to estimate a planning path;

a shared controller (170), electrically connected to the first robot (110) and the second robot (120), and configured to adjust a collaborative rate of the first robot (110) to the second robot (120) by regulating a shared weighting according to the position of the second robot (120) and the planning path; and

a computer calculation unit (180), electrically connected to the first robot (110), the second robot (120), the needle insertion device (130) and the optical tracker (160).

2. The compound robotic system according to claim 1, wherein the needle insertion device (130) comprises:

a guide slot (131);

a puncture needle (132), located in the guide slot (131); and

a sensor, configured to sense a force and tactile feedback of the puncture needle (132) when passing through a muscle tissue of the patient.

3. The compound robotic system according to any one of claims 1-2, wherein the second robot (120) comprises:

a base (121), connected to one end of the first robot (110) away from the carrying platform, wherein the first robot (110) is configured to control a position of the base (121);

a moving platform (122), connected to the needle insertion device (130) and the first optical positioning ball module (140); and

a plurality of moving links (123), located between the base (121) and the moving platform (122), and each having a plurality of joints so that the second robot (120) has a plurality of degrees of freedom.

4. The compound robotic system according to claim 3, wherein the second robot (120) is configured to control an entry attitude of the needle insertion device (130), and the shared controller (170) is configured to share a target attitude of the first robot (110) and a target attitude of the second robot (120).

5. The compound robotic system according to claim 4, wherein the target attitude of the first robot (110) conforms to a following formula:

$$\left( {}^{Path}_{B_d}X \right) = {}^{Path}_{B}X + w \left( {}^{Path}_{t_d}X - {}^{Path}_{t}X \right)$$

wherein ${}^{Path}_{t_d}X$ is a target attitude of a puncture needle (132) of the needle insertion device (130) relative to the planning path, ${}^{Path}_{t}X$ is a current attitude of the puncture needle (132) relative to the planning path, ${}^{Path}_{B}X$ is a current attitude of the base (121) of the second robot (120) relative to the planning path, ${}^{Path}_{B_d}X$ is a target attitude of the base (121) of the second robot (120) relative to the planning path, and w is the shared weighting.

6. The compound robotic system according to claim 5, wherein the target attitude of the second robot (120) conforms to a following formula:

$${}^{B_d}_{t_d}T = {}^{path}_{B_d}T^{-1} {}^{Path}_{t_d}T$$

wherein ${}^{path}_{B_d}T$ is a target attitude to be achieved by the base (121) of the second robot (120) relative to the planning

path, $_{B_d}^{path}T^{-1}$ is an inverse matrix of $_{B_d}^{path}T$ and $_{t_d}^{B_d}T$ is an attitude of the needle insertion device (130) relative to the base (121) when the second robot (120) completes alignment of an entry path with the planning path.

7. The compound robotic system according to any one of claims 4-6, wherein the target attitude of the first robot (110) conforms to a following formula:

$$_{B_d}^{Path}T = {_t^{Path}T}\,_{B_d}^{t}T$$

wherein $_{B_d}^{Path}T$ is the target attitude of the first robot (110) and $_t^{Path}T$ is a current attitude of a puncture needle (132) of the needle insertion device (130) relative to the planning path, and the target attitude $_{B_d}^{Path}T$ of the first robot (110) is planned according to the current attitude $_t^{Path}T$ of the puncture needle (132) and a working space of the second robot (120), so that $_{B_d}^{t}T$ meets requirements of the working space of the second robot (120).

8. The compound robotic system according to claim 7, wherein the target attitude of the second robot (120) conforms to a following formula:

$$_{t_d}^{B}T = {_B^{path}T^{-1}}\,_{t_d}^{Path}T$$

wherein $_{t_d}^{B}T$ is the target attitude of the second robot (120), $_B^{path}T^{-1}$ is an path path inverse matrix of $_B^{path}T$, $_B^{path}T$ is a current attitude of the base (121) of the second robot (120) relative to the planning path, and $_{t_d}^{Path}T$ is an attitude of the needle insertion device (130) relative to the planning path when the second robot (120) completes alignment of an entry path with the planning path.

9. The compound robotic system according to any one of claims 1-8, wherein the shared controller (170) is configured to adjust the shared weighting according to a tracking error of the optical tracker (160) for the first optical positioning ball module (140).

10. The compound robotic system according to any one of claims 1-9, further comprising:

a second optical positioning ball module (190), located on the first robot (110), wherein
the optical tracker (160) is configured to sense the second optical positioning ball module (190) and then to obtain a reference coordinate.

11. The compound robotic system according to claim 10, wherein the optical tracker (160) is configured to measure movement of the skin markers (150) to estimate a respiratory motion of the patient, the computer calculation unit (180) is configured to create a respiratory model based on the respiratory motion, and the respiratory model is configured to estimate an instant position of a lesion of the patient.

12. The compound robotic system according to claim 11, wherein the computer calculation unit (180) is configured to obtain the reference coordinate from the second optical positioning ball module (190) and to filter out the respiratory motion of the patient to track a hard tissue motion of the patient.

13. The compound robotic system according to claim 12, wherein the hard tissue motion of the patient obtains a coordinate of movement of the patient movement by means of an iterative closest point (ICP).

14. The compound robotic system according to any one of claims 11-13, wherein the respiratory model conforms to a following formula:

$$\begin{bmatrix} x_i(t) \\ y_i(t) \\ z_i(t) \end{bmatrix} = \begin{bmatrix} a_1 x_i(t-1) + a_2 x_i(t-2) + \cdots + a_k x_i(t-k) \\ b_1 y_i(t-1) + b_2 y_i(t-2) + \cdots + b_k y_i(t-k) \\ c_1 z_i(t-1) + c_2 z_i(t-2) + \cdots + c_k z_i(t-k) \end{bmatrix}$$

wherein $i$ is an index of the skin markers (150), $x_i$, $y_i$ and $z_i$ are x, $y$ and z coordinates of the skin markers (150) respectively, t is a time, k is the number of past moments used, and $a_1$-$a_k$, $b_1$-$b_k$ and $c_1$-$c_k$ are coefficients calculated from an autoregression model.

15. The compound robotic system according to claim 14, wherein the respiratory model is configured to instantly estimate positions of the skin markers (150) for a next time.

16. The compound robotic system according to any one of claims 11-15, wherein the respiratory motion of the skin markers (150) outside and a position of the lesion inside are configured to create an outside-inside correlation model to estimate an instant position of the lesion, the outside-inside correlation model being calculated using a linear regression.

FIG. 1

FIG. 2

FIG. 3A

FIG. 3B

FIG. 4

FIG. 5

EP 4 699 563 A1

FIG. 6

FIG. 7

FIG. 8

FIG. 9

**EUROPEAN SEARCH REPORT**

Application Number

EP 25 19 0143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/378526 A1 (BALICKI MARCIN ARKADIUSZ [US] ET AL) 1 December 2022 (2022-12-01) * paragraphs [0079] - [0083], [0089] - [0126]; figures 1-6 * ----- | 1-16 | INV. A61B34/30 ADD. A61B34/20 A61B90/00 |
| A | US 2016/249990 A1 (GLOZMAN DANIEL [IL] ET AL) 1 September 2016 (2016-09-01) * paragraphs [0021], [0022], [0058] - [0063]; figures 1-8 * ----- | 1-16 | |
| A | SMOLJKIC GABRIJEL ET AL: "Control of a hybrid robotic system for computer-assisted interventions in dynamic environments", INTERNATIONAL JOURNAL OF COMPUTER ASSISTED RADIOLOGY AND SURGERY, SPRINGER, DE, vol. 11, no. 7, 11 December 2015 (2015-12-11), pages 1371-1383, XP035990796, ISSN: 1861-6410, DOI: 10.1007/S11548-015-1333-8 [retrieved on 2015-12-11] * the whole document * ----- | 1-16 | |
| A | KAPOOR A ET AL: "Suturing in confined spaces: constrained motion control of a hybrid 8-DoF robot", ADVANCED ROBOTICS, 2005. ICAR '05. PROCEEDINGS., 12TH INTERNATIONAL CONFERENCE ON SEATLE, WA, USA JULY 18-20, 2005, PISCATAWAY, NJ, USA,IEEE, 18 July 2005 (2005-07-18), pages 452-459, XP010835313, DOI: 10.1109/.2005.1507449 ISBN: 978-0-7803-9178-9 * the whole document * ----- | 1-16 | |

TECHNICAL FIELDS SEARCHED (IPC)

A61B

-/--

The present search report has been drawn up for all claims

| Place of search | Date of completion of the search | Examiner |
|---|---|---|
| The Hague | 18 December 2025 | Viidebaum, Mikk |

EPO FORM 1503 03.82 (P04C01)

page 1 of 2

Europäisches
Patentamt

European
Patent Office

Office européen
des brevets

**EUROPEAN SEARCH REPORT**

Application Number

EP 25 19 0143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | PEIHAN ZHANG ET AL: "Dexterous Control of an 11-DOF Redundant Robot for CT-Guided Needle Insertion With Task-Oriented Weighted Policies", ARXIV.ORG, CORNELL UNIVERSITY LIBRARY, 201 OLIN LIBRARY CORNELL UNIVERSITY ITHACA, NY 14853, 18 March 2025 (2025-03-18), XP091963034, * the whole document * | 1-16 | |

TECHNICAL FIELDS
SEARCHED      (IPC)

The present search report has been drawn up for all claims

| Place of search | Date of completion of the search | Examiner |
|---|---|---|
| The Hague | 18 December 2025 | Viidebaum, Mikk |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 699 563 A1**

## ANNEX TO THE EUROPEAN SEARCH REPORT
## ON EUROPEAN PATENT APPLICATION NO.

EP 25 19 0143

18-12-2025

| Patent document cited in search report | Publication date | Patent family member(s) | Publication date |
|---|---|---|---|
| US 2022378526 A1 | 01-12-2022 | CN 114650785 A | 21-06-2022 |
| | | EP 3824839 A1 | 26-05-2021 |
| | | EP 4054468 A1 | 14-09-2022 |
| | | JP 2023500464 A | 06-01-2023 |
| | | US 2022378526 A1 | 01-12-2022 |
| | | WO 2021089550 A1 | 14-05-2021 |
| US 2016249990 A1 | 01-09-2016 | CA 2926621 A1 | 16-04-2015 |
| | | CN 105813585 A | 27-07-2016 |
| | | EP 3054868 A1 | 17-08-2016 |
| | | JP 6581973 B2 | 25-09-2019 |
| | | JP 2016536047 A | 24-11-2016 |
| | | US 2016249990 A1 | 01-09-2016 |
| | | US 2020179066 A1 | 11-06-2020 |
| | | US 2022280251 A1 | 08-09-2022 |
| | | WO 2015052719 A1 | 16-04-2015 |

EPO FORM P0459